Europäisches Patentamt

European Patent Office

Office européen des brevets

(11) Veröffentlichungsnummer: **0 320 728**
**A2**

(12) **EUROPÄISCHE PATENTANMELDUNG**

(21) Anmeldenummer: 88120215.4

(22) Anmeldetag: 03.12.88

(51) Int. Cl.⁴: **C07C 45/86 , C07C 45/82 , C07C 47/02**

(30) Priorität: 12.12.87 DE 3742254

(43) Veröffentlichungstag der Anmeldung:
21.06.89 Patentblatt 89/25

(84) Benannte Vertragsstaaten:
AT BE CH DE ES FR GB IT LI NL SE

(71) Anmelder: HOECHST AKTIENGESELLSCHAFT
Postfach 80 03 20
D-6230 Frankfurt am Main 80(DE)

(72) Erfinder: Weber, Jürgen, Dr. Dipl.-Chem.
Bunsenstrasse 17
D-4200 Oberhausen 11(DE)
Erfinder: Kampmann, Detlef, Dr. Dipl.-Chem.
Rankenweg 3
D-4630 Bochum 6(DE)
Erfinder: Kniep, Claus
Rosenstrasse 93
D-4200 Oberhausen 1(DE)
Erfinder: Zgorzelski, Wolfgang
Thüringerstrasse 20
D-4200 Oberhausen 11(DE)

(54) **Verfahren zur Stabilisierung von Aldehyden.**

(57) Zur Stabilisierung gegenüber Polymerisation und Autokondensation werden Aldehyde erfindungsgemäß unter Zusatz von Triethanolamin destilliert.

EP 0 320 728 A2

## Verfahren zur Stabilisierung von Aldehyden

Die vorliegende Erfindung betrifft ein Verfahren zur Stabilisierung von Aldehyden gegen Polymerisations- und Autokondensationsreaktionen.

Aliphatische Aldehyde neigen aufgrund ihrer großen Reaktivität zur Polymerisation und Autokondensation. Die Polymerisation führt unter starker Wärmeentwicklung zu acetalartigen, höhermolekularen Verbindungen, die sowohl Ring- als auch Kettenstruktur besitzen können. Sie wird durch saure Reagenzien wie Schwefelsäure, Salzsäure, Fluorwasserstoff, Bortrifluorid oder Aluminiumchlorid katalysiert. Selbst die durch Oxidation der Aldehyde in Gegenwart von Luftsauerstoff entstehenden, geringen Carbonsäuremengen sind für die Polymerbildung ausreichend. Darüber hinaus begünstigen tiefe Temperaturen, d.h. Temperaturen um den Gefrierpunkt und darunter, die Polymerisation der aliphatischen Aldehyde.

Die kettenförmigen Polyaldehyde sind Gemische verschiedener Polymerhomologer. Sehr gut untersucht sind die linearen Aldehydpolymeren des Formaldehyds. Von ihm existieren sowohl Dimere als auch niedermolekularer Paraformaldehyd und hochmolekularer Polyformaldehyd (Polyoxymethylen) mit einer Molmasse von über 100.000 g/mol.

Die cyclischen Polymere sind meist trimer und besitzen Trioxan-Struktur. So bildet Formaldehyd z.B. Trioxymethylen (1,3,5-Trioxan) und Acetaldehyd den flüssigen Paraaldehyd (2,4,6-Trimethyl-1,3,5-trioxan).

Aldehyde, die eine zur Carbonylgruppe alpha-ständige Methyl- oder Methylengruppe enthalten, dimerisieren unter dem katalytischen Einfluß von Basen oder Säuren zu Aldolen (Aldoladdition). Meist bleibt die Reaktion nicht auf der Stufe des Additionsproduktes stehen, sondern es erfolgt Wasserabspaltung.

Wegen des Überganges in höhermolekulare Verbindungen können Aldehyde nicht beliebig lange Zeit aufbewahrt werden. Zwar zerfallen die Polymerisations- und Autokondensationsprodukte der Aldehyde häufig bei höherer Temperatur, doch steht ihre Bildung einer uneingeschränkten technischen Verwendung der Aldehyde entgegen.

Über einen begrenzten Zeitraum können Polymerisation und Autokondensation der Aldehyde dadurch verhindert werden, daß man sie in hochreiner Form herstellt. Die hierzu erforderlichen Reinigungsoperationen sind jedoch so aufwendig, daß sie sich aus wirtschaftlichen Gründen meist verbieten.

Ein anderer Weg, Polymerisations- und Autokondensationsreaktionen zu unterbinden besteht darin, den Aldehyden inhibierend wirkende Stoffe zuzusetzen. An diese Stoffe stellt die Praxis eine Reihe Forderungen, die erfüllt sein müssen, falls der Aldehyd ohne Einschränkung den verschiedensten Anwendungsfällen zugeführt werden soll. Hierzu gehört, daß die betreffende Substanz bereits in niedriger Konzentration lange Zeit hindurch wirksam ist und darüber hinaus bei der Verarbeitung des Aldehyds durch chemische Umsetzungen nicht stört.

Als Stabilisatoren beschrieben sind z.B. Mercaptobenzimidazol und 2,2-Methylen-di-(4-methyl-6-tert.-butylphenol). Sie werden eingesetzt, Polymerenbildung aus Isobutyraldehyd zu vermeiden, sind aber nur unzureichend lange Zeit wirksam.

Nach einem weiteren Verfahren setzt man Aldehyden zur Verhinderung der Polymerisation eine Lösung von Diphenylamin und Ethanol zu. Auch diese Arbeitsweise stellt nicht sicher, daß die Polymerisation über einen längeren Zeitraum unterdrückt wird.

Sehr gut bewährt hat sich die Stabilisierung von gesättigten, aliphatischen Aldehyden mit 3 bis 14 Kohlenstoffatomen durch Zusatz von Triethanolamin oder Dimethylethanolamin nach DE-C2 29 17 789. Diese Stabilisatoren sind in sehr geringer Konzentration wirksam, bereits 10 Gew.-ppm der Amine (bezogen auf den Aldehyd) schließen die Bildung hochmolekularer Verbindungen über einen Zeitraum von mehreren Wochen aus. In Konzentrationen von 20 bis 100 Gew.-ppm (bezogen auf den Aldehyd) unterbinden sie die Bildung von Polymeren bzw. Autoaldolisierungsprodukten bei der Lagerung der Aldehyde selbst bei niedrigen Temperaturen, ohne weitere Vorkehrungen über einen Zeitraum von mehreren Monaten.

Wegen ihrer hohen Wirksamkeit und der Möglichkeit, sie daher in sehr geringen Konzentrationen anzuwenden, werden die Stabilisatoren bei der Weiterverarbeitung der Aldehyde im allgemeinen toleriert. In Ausnahmefällen werden aber Aldehyde gefordert, die nicht nur frei von Polymerisations-und Autokondensationsprodukten sind, sondern auch von Zusätzen, die ihr Langzeitverhalten verbessern.

Es bestand somit die Aufgabe, ein Verfahren zu entwickeln, das die Stabilisierung von Aldehyden ohne Zusatz von Stoffen, die in den Aldehyden verbleiben, ermöglicht.

Die Erfindung besteht in einem Verfahren zur Stabilisierung aliphatischer Aldehyde mit 2 bis 12, insbesondere 2 bis 8 Kohlenstoffatomen im Molekül. Es ist dadurch gekennzeichnet, daß man den Aldehyd unter Zusatz von Triethanolamin destilliert.

Überraschenderweise hat sich gezeigt, daß es nicht erforderlich ist, den Aldehyden nach dem Verfahren der DE-C2 29 17 789 Triethanolamin zuzusetzen, um die Bildung polymerer Verbindungen wirksam zu verhindern. Es reicht vielmehr aus, die Aldehyde in Gegenwart von Triethanolamin zu destillieren. Durch diese Maßnahme wird nicht nur sichergetellt, daß die Aldehyde keine Polymerisations- und Autokondensationsprodukte enthalten und über Monate hinweg auch nicht bilden. Man erhält zudem Aldehyde, die frei von Fremdstoffen sind und daher ohne Einschränkung den verschiedensten Umsetzungen und Verwendungsarten zugeführt werden können. Voraussetzung ist lediglich, die Aldehyde so zu lagern, daß ein Zutritt von Fremdstoffen ausgeschlossen ist. Die Lagerungstemperatur hat bei den nach dem erfindungsgemäßen Verfahren behandelten Aldehyden kaum Einfluß auf das Polymerisations- bzw. Kondensationsverhalten. Auch in der Kälte, die, wie oben bereits gesagt wurde, ihre Polymerisationsneigung erhöht, bleiben die Aldehyde als monomere Verbindungen erhalten.

Worauf die Wirksamkeit des Zusatzes von Triethanolamin bei der Aldehyddestillation beruht, wurde im einzelnen nicht untersucht. Es gibt jedoch deutliche Hinweise darauf, daß das Triethanolamin u.a. sauer reagierende Substanzen, die in geringer Menge in den Aldehyden enthalten sind, bindet. Solche Stoffe, sie können z.B. bei der Herstellung in die Aldehyde gelangt sein, wirken als Polymerisations- oder Kondensationskatalysatoren.

Beispiele für Aldehyde mit 2 bis 12, insbesondere 2 bis 8 Kohlenstoffatomen, die nach der neuen Arbeitsweise stabilisiert werden können, sind Propanal, n-Butanal, i-Butanal, n-Pentanal, n-Hexanal, n-Octanal, Dimethylhexanal. Auch bei höheren Aldehyden mit mehr als acht Kohlenstoffatomen, wie die Nonylaldehyde, Decylaldehyde, Undecylaldehyde, ferner Laurinaldhyd, Methylnonylacetaldehyd, die in großem Umfang bei der Riechstoffherstellung Verwendung finden, läßt sich nach dem neuen Verfahren die Bildung höhermolekularer Verbindungen mit Erfolg unterbinden.

Das Triethanolamin wird in handelsüblicher Form, eingesetzt. Dem zu stabilisierenden Aldehyd wird es in einer Menge von 5 bis 10.000, insbesondere 10 bis 500 ppm, bezogen auf den Aldehyd, zugegeben.

Die Destillation der Aldehyde unter Zusatz von Triethanolamin gemäß der beanspruchten Arbeitsweise kann nach den in der chemischen Technik üblichen Verfahren erfolgen. Sie wird im allgemeinen bei Normaldruck, jedoch auch bei erhöhtem und, insbesondere bei höheren Aldehyden, auch unter vermindertem Druck vorgenommen.

In den folgenden Beispielen wird das neue Verfahren beschrieben. Es ist auf die hier dargestellten Ausführungsformen nicht beschränkt.

Beispiel 1 (Vergleich)

Über eine 1 m-Kolonne aus Edelstahl werden 1500 g technisches n-Butanal mit einem Gehalt von 38,3 % Trimeraldehyd unter Normaldruck destilliert. Es werden drei der Menge nach annähernd gleich große Fraktionen und 49,4 Gew.-% Rückstand erhalten. Sowohl die einzelnen Fraktionen als auch der Rückstand werden über einen Zeitraum von 10 Wochen unter gleichen Bedingungen gelagert und regelmäßig gaschromatografisch untersucht. Man erhält folgende Ergebnisse:

## Trimergehalt (GC) in Gew.%

| Wochen | 0 | 3 | 5 | 10 |
|---|---|---|---|---|
| 1. Fraktion | <0,01 | 0,02 | 0,04 | 0,57 |
| 2. Fraktion | <0,01 | 0,03 | 0,16 | 3,07 |
| 3. Fraktion | <0,01 | 0,09 | 0,38 | 6,80 |
| Rückstand | 78,98 | 79,14 | 79,62 | 79,20 |

Beispiel 2

1500 g des in Beispiel 1 eingesetzten n-Butanals werden unter Zugabe von 20 Gew.-ppm Triethanolamin destilliert. Das Destillationsprodukt wird, wie in Beispiel 1, über einen Zeitraum von 10 Wochen gelegert

und regelmäßig gaschromatographisch untersucht. Die Ergebnisse sind in der nachstehenden Tabelle zusammengefaßt.

## Trimergehalt (GC) in Gew.-%

| Wochen | 0 | 3 | 5 | 10 |
|---|---|---|---|---|
| 1. Fraktion | <0,01 | <0,01 | <0,01 | <0,01 |
| 2. Fraktion | <0,01 | <0,01 | <0,01 | 0,06 |
| 3. Fraktion | <0,01 | <0,01 | <0,01 | 0,09 |
| Rückstand | 77,89 | 77,81 | 78,50 | 78,59 |

Das gesamte Triethanolamin verbleibt im Rückstand

## Ansprüche

1.) Verfahren zur Stabilisierung von Aldehyden mit 2 bis 12, insbesondere 2 bis 8 Kohlenstoffatomen im Molekül, dadurch gekennzeichnet, daß man sie unter Zusatz von Triethanolamin destilliert.

2.) Verfahren nach Anspruch 1, dadurch gekennzeichnet, daß man unter Zusatz von 5 bis 10.000, insbesondere von 10 bis 500 Gew.-ppm Triethanolamin destilliert.